Europäisches Patentamt

European Patent Office

Office européen des brevets

(11) Numéro de publication: **0 306 423**
**A1**

## (12) DEMANDE DE BREVET EUROPEEN

(21) Numéro de dépôt: **88420299.5**

(22) Date de dépôt: **01.09.88**

(51) Int. Cl.⁴: **A 61 K 9/18**
**A 61 K 31/52**

(30) Priorité: **02.09.87 FR 8712707**

(43) Date de publication de la demande:
**08.03.89 Bulletin 89/10**

(84) Etats contractants désignés:
**AT BE CH DE ES FR GB GR IT LI LU NL SE**

(71) Demandeur: **MEDIBREVEX, Société Civile dite**
**1 avenue Marcelin Berthelot**
**F-38100 Grenoble (FR)**

(72) Inventeur: **Clairet Ayache, Josiane**
**3 boulevard Joffre**
**F-38000 Grenoble (FR)**

**Ayache, Jean-Jacques**
**3 boulevard Joffre**
**F-38000 Grenoble (FR)**

**Bruttmann, Georges**
**11 Place Victor Hugo**
**F-38000 Grenoble (FR)**

**Pedrali, Patrick**
**Chemin des Chapelaines La Boutaevilloise**
**F-74000 Annecy (FR)**

**Robert, Serge**
**14 rue des Dêgues**
**B-1440 Braine le Chateau (BE)**

(74) Mandataire: **Maureau, Philippe et al**
**Cabinet Germain & Maureau Le Britannia - Tour C 20, bld**
**Eugène Déruelle Boîte Postale 3011**
**F-69392 Lyon Cédex 03 (FR)**

(54) Nouvelles formes galéniques de théophylline pour administration par voie per- et sublinguale.

(57) Selon l'invention, la théophylline est continue, en quantités strictement contrôlées et reproductibles, dans des supports solides prévus pour une libération progressive du principe actif par voie per- et sublinguale.

Le procédé de préparation des nouvelles formes galéniques de théophylline comporte les étapes suivantes :
- mise en solution de la théophylline dans un solvant polaire pour obtenir une solution mère ;
- préparation de dilutions à différentes concentrations,
- fractionnement de chacune de ces dilutions en sous-dilutions,
- imprégnation par multi-imprégnation ou imprégnation fractionnée d'un support solide pharmaceutiquement acceptable avec chacune des sous-dilutions, chacune desdites étapes d'imprégnation étant suivie d'un séchage à air forcé et desséché à une température inférieure ou égale à 30°C ;
- imprégnation finale protectrice du type dragéification.

Le support solide peut également être un gel préparé à partir d'une solution aqueuse de théophylline ou de l'un de ses sels solubles.

EP 0 306 423 A1

**Description**

# NOUVELLES FORMES GALENIQUES DE THEOPHYLLINE POUR ADMINISTRATION PAR VOIE PER- ET SUBLINGUALE

La présente invention concerne de nouvelles formes galéniques de théophylline pour administration par voie per- et sublinguale.

On connaît tout l'intérêt que présentent les bronchodilatateurs dans le traitement des affections respiratoires dyspnéisantes et la toute première place que tient la théophylline dans la pharmacopée.

Les formes galéniques de théophylline utilisées le plus communément sont:
- les formes orales : comprimés et sirops,
- la forme rectale : suppositoires,
- la forme injectable,
- la forme soluble par aérosols.

L'activité clinique et l'utilité de la théophylline ne sont plus à démontrer. Un regain d'intérêt a été suscité par la mise au point de méthodes de dosages sériques simples.

Ces dosages sériques ont permis d'étudier la pharmacocinétique de la théophylline et de constater que la vitesse d'absorption dépend essentiellement de la forme galénique : elle est très rapide avec les solutions hydro-alcooliques (sirops), le pic sérique étant atteint entre 30 et 90 minutes : l'absorption est plus lente avec les formes micronisées, plus lente encore avec les autres formes.

Après la prise d'un comprimé de théophylline simple, le pic plasmatique est atteint en deux heures. Différentes formes permettent un véritable contrôle de la libération de la théophylline selon la structure même et l'enrobage des comprimés.

Par voie rectale, la résorption des suppositoires est plus lente et plus irrégulière car il existe de grandes variations individuelles.

La voie orale ou la voie rectale ne sont envisageables que si le patient n'a pas de maladie digestive associée. Dans le cas contraire, il est fréquent de devoir recourir aux injections intraveineuses qui doivent être lentes et sont souvent pénibles.

Les Inventeurs ont découvert, de façon surprenante, qu'il était possible d'administrer la théophylline par voie per- et sublinguale et que ces nouvelles formes galéniques présentaient des avantages importants comparativement aux formes galéniques existantes.

Les nouvelles formes galéniques de théophylline selon l'invention sont caractérisées en ce que la théophylline est contenue, en quantités strictement contrôlées et reproductibles, dans des supports solides prévus pour une libération progressive du principe actif par voie per- et sublinguale.

On a, en effet, déterminé que ces nouvelles formes galéniques permettaient un passage très rapide de la théophylline dans le courant sanguin, grâce au réseau vasculaire sub et perlingual, et ceci en évitant le passage digestif.

Cette voie per- et sublinguale est utilisée à cause de sa configuration anatomique qui en fait une entité par rapport aux autres éléments de la cavité buccale.

Schématiquement, on peut décrire la région sublinguale de la manière suivante (chacun des éléments constitutifs ayant des propriétés utiles dans l'administration des médicaments introduits par cette voie).

Globalement, la région sublinguale comprend:
- la face inférieure de la langue qui sert de plafond, très riche en vaisseaux sanguins : veines, artères et vaisseaux lymphatiques ;
- le plancher de la bouche qui constitue la partie inférieure de la région sublinguale. C'est également une région anatomique qui comporte beaucoup de vaisseaux sanguins et surtout un très riche réseau veineux, des artères et des vaisseaux lymphatiques ;
- les bords de la région sublinguale sont formés par les parties montantes du maxillaire inférieur, les gencives et les dents.

Dans le plancher de la bouche, on trouve des glandes salivaires, les glandes sublinguales, qui secrètent de la salive dès qu'il existe un contact avec la région sublinguale. Par sa composition, la salive participe activement au délitement du produit pharmacologiquement actif présenté sous cette forme galénique sublinguale particulière selon l'invention.

Par ailleurs, la structure histologique montre l'existence de cellules immuno-compétentes en grand nombre qui vont, dans certains cas, favoriser l'activité médicamenteuse.

Par rapport aux autres constituants de la bouche : intérieur des joues, partie supérieure de la langue, la région sublinguale apparaît donc comme une entité anatomique particulière qui en fait une voie d'administration médicamenteuse privilégiée comparable à l'administration des médicaments par voie injectable.

Tous les autres médicaments dont le délitement se fait dans la bouche sont forcément avalés.

Dans la forme galénique sublinguale selon l'invention, l'activité du produit est fondée sur sa possibilité d'absorption rapide sans déglutition. L'avantage que présente cette forme galénique par rapport aux autres formes galéniques utilisées à l'intérieur de la cavité buccale est que le produit passe directement dans le sang, ce qui évite le métabolisme hépatique ; le produit agit donc plus rapidement avec une efficacité maximale.

Outre le dosage plasmatique de la théophylline qui confirme la biodisponibilité et une bonne cinétique du produit, on a pu juger de l'amélioration clinique, d'une part, sur les symptomes cliniques et, d'autre part, sur une courbe spirographique en particulier le V.E.M.S. ou volume expiratoire maximum seconde (ou F.E.V.I. des anglo-saxons).

L'absorption de ces nouvelles formes galéniques n'est pas traumatisante et le malade n'a plus besoin d'une aide para-médicale.

La posologie est, bien évidemment, adaptable selon les besoins.

D'autre part, en dehors des incompatibilités médicamenteuses connues, il est possible, avec cette nouvelle forme galénique, d'associer la théophylline à d'autres médicaments.

Cette forme galénique ne s'adresse qu'aux traitements ambulatoires et en aucun cas, la voie sub-linguale ne pourra se substituer à un traitement intensif. Cependant, cette forme galénique sub-linguale, d'action rapide, d'administration simple, évite les abus et permet un excellent contrôle de l'obstruction bronchique sans avoir recours à une injection intraveineuse souvent redoutée par les patients.

Le procédé d'obtention de cette nouvelle forme galénique selon l'invention va être exposé en détail.

Le produit de départ est la théophylline qui est lyophilisée.

Le lyophilisat obtenu est mis en solution dans tout solvant convenable, de façon à obtenir une solution mère. Le solvant utilisé peut être l'eau ou sérum physiologique : il peut également être choisi parmi d'autres solvants, de préférence polaires, tel que l'alcool éthylique de titre faible.

En cas de dilutions de la solution mère, toutes les précautions sont prises pour que la quantité de solvant reste constante, ceci afin de réaliser toutes les imprégnations de façon identique.

On réalise, ensuite, dans une turbine, à l'aide d'un injecteur du type dénommé "spray-doseur", l'imprégnation, à l'aide de chacune des dilu tions, de globules constitués de façon connue en soi, d'un support solide pharmaceutiquement acceptable et spécialement d'un mélange saccharose-lactose. Il est évident que, sans sortir du cadre de l'invention, ces globules pourraient être remplacés par des poudres ou des comprimés, également adaptés à l'application par voie perlinguale ou sublinguale.

La technique d'imprégnation est celle de la multi-imprégnation ou de l'impregnation fractionée de manière à garantir une parfaite répartition du principe actif homogène.

Entre chaque imprégnation, le séchage, effectué à une température inférieure ou égale à 30°C, est réalisé par passage d'air forcé et desséché dans une chambre où règne une dépression obtenue par la différence du débit existant entre ce passage d'air et un puissant extracteur.

La dernière imprégnation est une imprégnation protectrice du type "dragéification.

Selon un autre mode de réalisation du procédé selon l'invention, et afin de garantir que soit totalement maintenue l'intégrité physico-chimique du principe actif, les différentes étapes de l'imprégnation sont effectuées sous atmosphère d'azote.

La dernière opération consiste à mettre en gélules les globules ainsi obtenus, ou éventuellement à placer lesdits globules ou les poudres ou comprimés dans des tubes-doses et à terminer le conditionnement de façon classique (blisters pour les gélules, coffrets pour les tubes-doses).

Les Inventeurs ont pu, par ailleurs, déterminer que le véhicule renfermant le ou les principes actifs pouvait aussi se présenter sous forme d'un gel oral soluble, dont la composition peut varier, et qui sera, par exemple, une cellulose microcristalline associée ou non à de la carboxyméthylcellulose sodique.

L'intérêt de cette forme est de pouvoir incorporer une concentration plus grande de principe actif que dans un comprimé perlingual, tout en maintenant une dissolution rapide du produit au niveau sublingual.

Le gel est présenté en emballage individuel mono-dose, assurant précision du dosage et sécurité d'emploi.

Dans ce cas, la théophylline (ou l'un de ses gels solubles) est mise en solution aqueuse.

La quantité de théophylline est fonction de la concentration finale souhaitée en principe actif. C'est la solution ainsi préparée qui est ensuite incorporée au gel fabriqué, d'autre part.

Différents types de traitement peuvent être proposés :
- traitement quotidien : prise de deux à six doses perlinguales à adapter selon les symptomes cliniques et la spirographie ;
- traitement occasionnel : prise de deux à quatre doses ou capsules lors d'une crise.

Les résultats cliniques sont très surprenants par rapport aux autres formes galéniques, pour les traitements de pratique quotidienne et cela sans avoir besoin du recours d'une aide para-médicale.

Une autre avantage surprenant est la rapidité d'action de la nouvelle forme galénique qui permet au malade de contrôler lui-même et rapidement son obstruction bronchique.

Les concentrations en théophylline peuvent, bien entendu, être adaptées sur demande ; de même, le support saccharose-lactose peut être remplacé par un autre support pharmaceutiquement acceptable adapté.

## Revendications

1- Nouvelles formes galéniques de théophylline, caractérisées en ce que la théophylline est contenue, en quantités strictement contrôlées et reproductibles, dans des supports solides prévus pour une libération progressive du principe actif par voie per- et sublinguale.

2- Nouvelles formes galéniques de théophylline selon la revendication 1, caractérisées en ce que le support solide est un gel.

3- Procédé de préparation de formes galéniques solides de théophylline selon la revendication 1, caractérisé par les étapes suivantes : - mise en solution de la théophylline dans un solvant polaire pour obtenir une solution mère ;
- preparation de dilutions à différentes concentrations,
- fractionnement de chacune de ces dilutions en sous-dilutions,
- imprégnation par multi-imprégnation ou imprégnation fractionée d'un support solide pharmaceutiquement acceptable avec chacune des sous-dilutions, chacune desdites étapes d'imprégnation étant suivie d'un séchage à air forcé et desséché à une température inférieure ou égale à 30°C ;
- imprégnation finale protectrice du type dragéification.

4- Procédé selon la revendication 3, caractérisé en ce que la théophylline se trouve sous forme lyophilisée.

5- Procédé selon la revendication 3 est la revendication 4, caractérisé en ce que le solvant polaire est choisi parmi l'eau, le sérum physiologique et l'alcool éthylique de titre faible.

6- Procédé selon l'une quelconque des revendications 3 à 5, caractérisé en ce que les différentes étapes de l'imprégnation sont effectuées sous atmosphère d'azote.

7- Procédé de préparation de gel selon la revendication 2 à partir de solution aqueuse de théophylline ou l'un de ses sels solubles incorporé dans un gel.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 219 458  (WARNER-LAMBERT CO.)<br>* Page 5, ligne 34; page 8, lignes 18-33; revendications 1,7,21 * | 1 | A 61 K   9/18<br>A 61 K  31/52 |
| Y | | 2-7 | |
| Y | G. NETIEN et al.: "Galenica", vol. 16, "Médicaments Homéopathiques", édition 2, 1986, pages 77-99, chapitre 2, Technique et Documentation, Paris, FR; "Fabrication du médicament homéopathique"<br>* Page 85, lignes 9-25; page 87, ligne 12 - page 88, ligne 4; page 94, lignes 1-10,35-43; page 97, ligne 21 - page 98, ligne 7 * | 2-7 | |
| A | CH-A- 156 609  (CHEM. PHARM. LABORATORIUM "BIKA" V. PÖHLMANN & APOTHEKER R. NAGEL)<br>* Page 2, colonne de gauche, ligne 24 - colonne de droite, ligne 35 * | | |
| A | FR-A-2 285 896  (NIPPON KAYAKU K.K.)<br>* Page 4, ligne 10 - page 5, ligne 17; revendications 1,4,7 * | | DOMAINES TECHNIQUES RECHERCHES (Int. Cl.4)<br><br>A 61 K |

Le présent rapport a été établi pour toutes les revendications

| Lieu de la recherche | Date d'achèvement de la recherche | Examinateur |
|---|---|---|
| LA HAYE | 10-11-1988 | TZSCHOPPE, D.A. |